# EUROPEAN PATENT APPLICATION

(11) **EP 4 528 278 A1**
(43) Date of publication of application: **26.03.2025**
(21) Application number: 23829596.8
(22) Date of filing: 10.04.2023
(51) Int. Cl.: G01N 33/558, G01N 33/543, G01N 33/53, G01N 21/78

(54) **SAMPLING DETECTION DEVICE**

(30) Priority: 30.06.2022 CN 202210758281
(71) Applicant: Jiangsu Bioperfectus Technologies Co., Ltd, Taizhou, Jiangsu 225300 (CN)
(72) Inventor: YANG, Biao, Taizhou, Jiangsu 225300 (CN); CHEN, Renyuan, Taizhou, Jiangsu 225300 (CN); WANG, Xin, Taizhou, Jiangsu 225300 (CN); JIN, Wei, Taizhou, Jiangsu 225300 (CN); LIU, Zhonghua, Taizhou, Jiangsu 225300 (CN); WANG, Guoqiang, Taizhou, Jiangsu 225300 (CN)
(74) Representative: Bayramoglu et al.
(86) International application number: PCT/CN2023/087291
(87) International publication number: WO 2024/001406

(57) **Abstract**

The present disclosure provides a sampling detection device, including an outer tube, an inner tube, and a tube cover, where the outer tube includes a reaction liquid holding chamber and a main body holding chamber; the reaction liquid holding chamber is communicated with the main body holding chamber; an end of the main body holding chamber is open; a side wall of the main body holding chamber includes a transparent structure; the inner tube includes a squeezing part and a test strip mounting part; the outer tube is configured to hold the inner tube; the test strip mounting part is located inside the main body holding chamber; the squeezing part is inserted into the reaction liquid holding chamber; the squeezing part cooperates with an inner wall of the reaction liquid holding chamber to form a gap; the tube cover is detachably connected to the outer tube; and the tube cover is configured to close or open an open side of the main body holding chamber. During sampling, a sampling swab is eluted in the outer tube, and the inner tube is placed into the outer tube. The squeezing part of the inner tube squeezes out a liquid in the reaction liquid holding chamber, such that the liquid immerses chromatography parts of multiple test strips. Then, result determination is conducted, thereby completing the multi-target detection. The present disclosure features simple structure, convenient operation, and high batch detection efficiency.

## Description

### TECHNICAL FIELD

The present disclosure relates to the technical field of biological detection, and in particular to a sampling detection device.

### BACKGROUND TECHNOLOGY

The principle of immunochromatography involves the use of a specific antibody. The specific antibody is fixed in a certain zone of a nitrocellulose membrane. When an end of the dry nitrocellulose is immersed in the sample, the sample moves forward along the membrane due to capillary action. When moved to the zone where the antibody is fixed, a corresponding antigen in the sample specifically binds to the antibody. By staining the zone with immunogold or immunoenzyme, a certain color is displayed, thereby enabling specific immunodiagnosis.

The Chinese patent application CN208705337U discloses a sampling detection device, which includes an upper card, a lower card, and test strips. The upper card is provided with ventilation holes, visual windows, and sampling holes. There are multiple sampling holes. The lower card is positioned opposite the upper card. A storage space is formed between the lower card and the upper card, and the storage space is communicated with the ventilation holes. The test strips are located within the storage space. There are multiple test strips spaced apart from each other. The test strips are opposite the visual windows, and each test strip is opposite at least one sampling hole.

When the sampling detection device in the prior art is used for detection, it requires the personnel to add the sample into at least one of the sampling holes, which is complex to operate and has poor batch detection efficiency. Therefore, it is necessary to improve the prior art.

### CONTENT OF THE INVENTION

In view of the defects in the prior art, an objective of the present disclosure is to provide a sampling detection device.

An embodiment of the present disclosure provides a sampling detection device, including an outer tube, an inner tube, and a tube cover, where the outer tube includes a reaction liquid holding chamber and a main body holding chamber arranged in sequence from bottom to top; the reaction liquid holding chamber is communicated with the main body holding chamber; an end of the main body holding chamber away from the reaction liquid holding chamber is open; a side wall of the main body holding chamber includes a transparent structure; the inner tube includes a squeezing part and a test strip mounting part connected in sequence; a peripheral side of the test strip mounting part is provided with multiple test strip mounting positions; the outer tube is configured to hold the inner tube; the test strip mounting part is located inside the main body holding chamber; the squeezing part is inserted into the reaction liquid holding chamber; the squeezing part cooperates with an inner wall of the reaction liquid holding chamber to form a gap that allows a reaction liquid to pass through; the tube cover is detachably connected to the outer tube; and the tube cover is configured to close or open an open side of the main body holding chamber.

Furthermore, an outer wall of the squeezing part is provided with a guide channel; and the guide channel extends from an end of the squeezing part away from the test strip mounting part towards the test strip mounting part.

Furthermore, the test strip mounting positions are recessed on an outer wall of the test strip mounting part, and an opening edge of each of the test strip mounting positions is provided with a limit stopper.

Furthermore, a pressing plate is provided in the tube cover; and after the tube cover is provided in place on the outer tube, the pressing plate abuts against the inner tube.

Furthermore, the inner tube includes a hollow structure; an upper end of the inner tube is open; and the inner tube includes an integrated structure or a split structure.

Furthermore, the test strip mounting part includes a mounting body and a connecting base; the mounting body and the connecting base are connected in an inserting manner; and an end of the connecting base away from the mounting body is securely connected to the squeezing part.

Furthermore, an outer wall of the squeezing part is provided with a guide channel; an end of the connecting base adjacent to the squeezing part is provided with an overflow port; the overflow port communicates the guide channel with an internal space of the inner tube; a side of the test strip mounting position located at the connecting base is provided with a first communicating hole; and the first communicating hole communicates the test strip mounting position with the internal space of the inner tube.

Furthermore, the test strip mounting part includes multiple mounting side plates; and each two adjacent mounting side plates are connected in a clamping manner.

Furthermore, a bottom of each of the mounting side plates is provided with a holding element; and multiple holding elements at a bottom of the test strip mounting part cooperate to hold the squeezing part.

Furthermore, a side of the test strip mounting position adjacent to the squeezing part is provided with a second communicating hole; and the second communicating hole communicates the test strip mounting position with an internal space of the inner tube.

### DESCRIPTION OF THE DRAWINGS

Other features, objectives, and advantages of the present disclosure will become more apparent by reading the detailed description of non-limiting embodiments with reference to the following drawings.
FIG. 1 is an overall exploded view of a sampling detection device according to Embodiment 1 of the present disclosure;
FIG. 2 is an overall section view of the sampling detection device according to Embodiment 1 of the present disclosure;
FIG. 3 is an overall section view of a sampling detection device according to Embodiment 2 of the present disclosure;
FIG. 4 is a top view of an inner tube according to Embodiment 2 of the present disclosure;
FIG. 5 is an overall exploded view of a sampling detection device according to Embodiment 3 of the present disclosure;
FIG. 6 is an isometric exploded view of the sampling detection device according to Embodiment 3 of the present disclosure; and
FIG. 7 is a top view of an inner tube structure according to Embodiment 3 of the present disclosure.

### Reference Numerals:

| | | | |
|---|---|---|---|
| 1. | outer tube | 6. | pressing plate |
| 11. | reaction liquid holding chamber | 71. | mounting body |
| 12. | main body holding chamber | 72. | connecting base |
| 2. | inner tube | 75. | overflow port |
| 21. | squeezing part | 76. | first communicating hole |
| 22. | guide channel | 81. | mounting side plate |
| 23. | test strip mounting part | 82. | clamping element |
| 3. | tube cover | 83. | clamping slot |
| 4. | test strip mounting position | 84. | holding element |
| 5. | limit stopper | 85. | second communicating hole |

### SPECIFIC IMPLEMENTATIONS

The present disclosure is described in detail below with reference to specific embodiments. The following embodiments will help those skilled in the art to further understand the present disclosure, without limiting the present disclosure in any way. It should be noted that several variations and improvements can also be made by a person of ordinary skill in the art without departing from the conception of the present disclosure. These all fall within the protection scope of the present disclosure.

### Embodiment 1

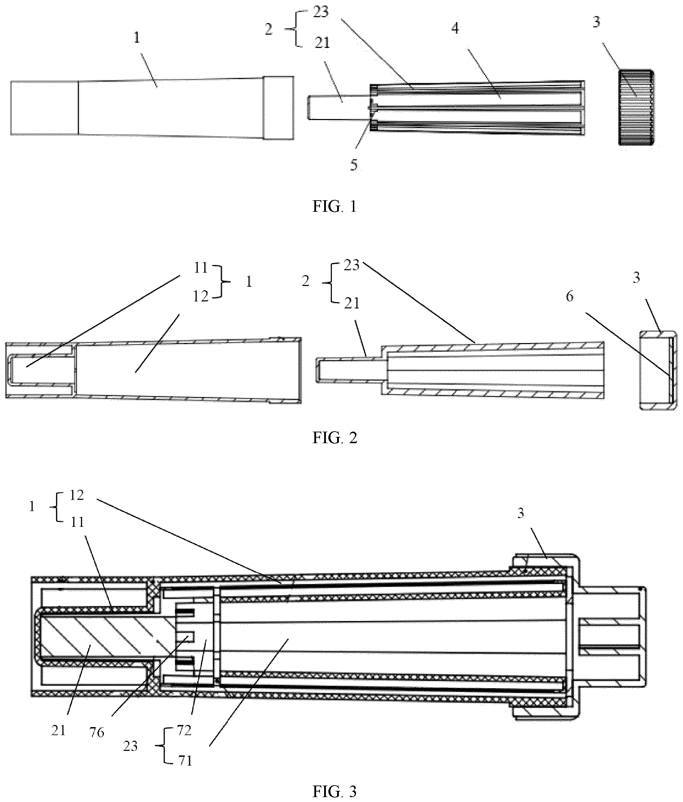

As shown in FIGS. 1 and 2, the present disclosure provides a sampling detection device, including outer tube 1, inner tube 2, and tube cover 3. The outer tube 1 is configured to hold a reaction liquid. An outer wall of the inner tube 2 is provided with multiple test strip mounting positions 4. The test strip mounting positions 4 are configured to securely mount test strips. During mounting, a chromatography part of the test strip is located at a side of the test strip mounting position 4 adjacent to a bottom of the inner tube 2. When multi-target detection is conducted, multiple test strips with different antibodies are provided at the multiple test strip mounting positions 4. A sampling swab is inserted into the outer tube 1, and the reaction liquid inside the outer tube 1 elutes the sampling swab. Then, place inner tube 2 into outer tube 1. A bottom structure of the inner tube 2 raises a liquid level inside the outer tube 1, causing the liquid inside the outer tube 1 to immerse the chromatography parts of the multiple test strips, thereby achieving multi-target detection. The design features a simple structure and is easy to operate.

Specifically, the outer tube 1 can be an integrated structure. The outer tube 1 includes reaction liquid holding chamber 11 and main body holding chamber 12 arranged in sequence from bottom to top. The reaction liquid holding chamber 11 is communicated with the main body holding chamber 12. An end of the main body holding chamber 12 away from the reaction liquid holding chamber 11 is open. A side wall of the main body holding chamber 12 includes a transparent structure. More specifically, the outer tube 1 is made of a transparent material.

The inner tube 2 can be an integrated structure. The inner tube 2 is a hollow structure. The upper end of the inner tube 2 is open. When multi-target detection is conducted, the staff places the eluted sampling swab into the inner tube 2 to prevent contamination of the environment by the sampling swab. The inner tube 2 includes squeezing part 21 and test strip mounting part 23 connected in sequence. A peripheral side of the test strip mounting part 23 is provided with the multiple test strip mounting positions 4. Each test strip mounting position 4 securely mounts one test strip. The test strip mounting positions 4 are recessed on an outer wall of the test strip mounting part 23, and limit stoppers 5 are integrally formed at an opening edge of the test strip mounting position 4.

The present disclosure is explained by taking the test strip mounting part 23 with eight test strip mounting positions 4 as an example. The eight test strip mounting positions 4 are arranged at equal intervals on the peripheral side of the test strip mounting part 23. The eight test strip mounting positions 4 are all in the shape of long strips. A length direction of the eight test strip mounting positions 4 is parallel to a length direction of the inner tube 2. The eight test strip mounting positions 4 have the same size. The limit stoppers 5 are integrally formed at opening edge positions at two width sides of each test strip mounting position 4. A length direction of each limit stopper 5 is parallel to the length direction of the inner tube 2, and each limit stopper 5 extends from the opening edge position of the test strip mounting position 4 to an upper side of the test strip mounting position 4. In this way, the test strip is securely mounted at the test strip mounting position 4, reducing the risk of accidental detachment of the test strip. When the eight test strips are provided at the eight test strip mounting positions 4, the test strips are at the same height.

As shown in FIGS. 1 and 2, the outer tube 1 is configured to hold the inner tube 2. The test strip mounting part 23 is located inside the main body holding chamber 12. The squeezing part 21 is inserted into the reaction liquid holding chamber 11. The squeezing part 21 cooperates with an inner wall of the reaction liquid holding chamber 11 to form a gap that allows the reaction liquid to pass through. The squeezing part 21 matches the reaction liquid holding chamber 11 in terms of shape, such that the squeezing part 21 can discharge all the liquid in the reaction liquid holding chamber 11 as much as possible. For example, both the squeezing part 21 and the reaction liquid holding chamber 11 are cylindrical, and the size of the squeezing part 21 is slightly smaller than that of the reaction liquid holding chamber 11, allowing the squeezing part 21 to be inserted into the reaction liquid holding chamber 11 and discharge the liquid therein. It should be clarified that the squeezing part 21 and the reaction liquid holding chamber 11 can have regular or irregular shapes, as long as the squeezing part 21 can be inserted into the reaction liquid holding chamber 11.

Furthermore, an outer wall of the squeezing part 21 may be provided with guide channel 22. The guide channel 22 extends from an end of the squeezing part 21 away from the test strip mounting part 23 towards the test strip mounting part 23. The guide channel 22 makes it easy to discharge the liquid inside the reaction liquid holding chamber 11.

More specifically, the tube cover 3 is detachably connected to the outer tube 1. The tube cover 3 is configured to close or open an open side of the main body holding chamber 12. The detachable connection can be a commonly used connection in the prior art, such as a threaded connection and an inserting connection. The tube cover 3 is embedded with pressing plate 6. After the tube cover 3 is provided in place on the outer tube 1, the pressing plate 6 abuts against the inner tube 2. The pressing plate 6 can be made of a rubber material. On the one hand, the design can prevent the liquid inside the outer tube 1 from flowing out from a connection between the outer tube 1 and the tube cover 3. On the other hand, the pressing plate 6 limits the axial direction of the inner tube 2, reducing the degree of freedom of movement of the inner tube 2 and facilitating the observation of the detection results by the staff.

The multi-target detection device of the present disclosure is particularly suitable for the following scenarios.
1. Respiratory swabs type. After sampling is completed, the sampling swab is eluted in the reaction liquid holding chamber 11 at the bottom of the outer tube, and then discarded into the inner tube 2. Then the inner tube 2 is put into the outer tube 1 to complete the chromatography. The type is applicable to common respiratory pathogen combinations such as influenza A (FLUA)/influenza B (FLUB)/respiratory syncytial virus (RSV)/adenovirus (ADV)/rubella virus (RV)/human parainfluenza viruses (HPIVs)/mycoplasma pneumoniae (MP)/2019 novel coronavirus (ncov-19).
2. Fecal samples type. After a fecal sample is collected with a sampling spoon, it is placed in the reaction liquid holding chamber 11 at the bottom of the outer tube. After thorough mixing, the inner tube is inserted into the outer tube 1 to complete the chromatography. The type is applicable to common combinations of multiple indicators such as human rotavirus (HRV)/ADV/Norovirus/helicobacter pylori (HP)/calprotectin (CALP)/occult blood (OB).
3. Blood and urine samples type. The liquid is collected and directly dripped into the reaction liquid holding chamber 11 at the bottom of the outer tube 1. After thorough mixing, the inner tube 2 is inserted into the outer tube 1 to complete the chromatography. The type is applicable to common blood-borne indicator detection.

The device is applied to various detection scenarios, achieving one-time sampling and rapid detection of multiple targets. The device is simple and fast to operate, and can obtain results in 15 min, achieving the effect of sample in and result out. The sampling waste is sealed in the detection device, avoiding contamination to the environment.

### Embodiment 2

Based on Embodiment 1, the present disclosure provides a sampling detection device with the inner tube 2 being a split structure.

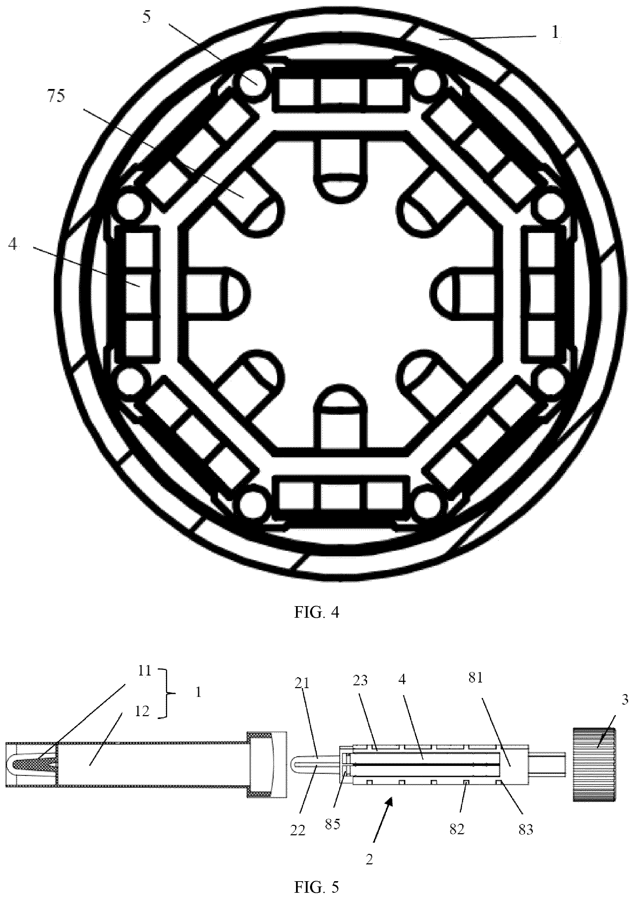

As shown in FIGS. 3 and 4, the test strip mounting part 23 includes mounting body 71 and connecting base 72. The mounting body 71 and the connecting base 72 are connected in an inserting manner. An end of the connecting base 72 away from the mounting body 71 is securely connected to the squeezing part 21. Specifically, an end of the connecting base 72 adjacent to the mounting body 71 is integrally formed with an inserting shaft, an end of the mounting body 71 adjacent to the connecting base 72 is provided with an inserting hole. There is one inserting hole between each two adjacent test strip mounting positions 4. The inserting shaft corresponds one-to-one with and is inserted in a matching manner into the inserting hole, achieving a split connection of the test strip mounting part 23. The design improves the mounting convenience of the test strip at the test strip mounting position 4, and features simple structure and easy manufacturing.

Furthermore, the inserting shaft includes a positioning section and a fixing section. The positioning section matches with the inserting hole to achieve positioning between the connecting base 72 and the mounting body 71. The fixing section matches with the inserting hole to achieve a secure connection between the connecting base 72 and the mounting body 71.

The outer wall of the squeezing part 21 is provided with the guide channel 22. An end of the connecting base 72 adjacent to the squeezing part 21 is provided with overflow port 75. The overflow port 75 communicates the guide channel 22 with an internal space of the inner tube 2. The liquid inside the outer tube 1 can flow into the internal space of the inner tube 2 along the guide channel 22 and the overflow port 75. A side of the test strip mounting position 4 located at the connecting base 72 is provided with first communicating hole 76. The first communicating hole 76 communicates the test strip mounting position 4 with the internal space of the inner tube 2, allowing the liquid to immerse the chromatography part of the test strip from two sides of the test strip, ensuring the chromatography effect and rate.

Specifically, when the implementation solution is used for respiratory swab type sampling detection, after the sampling swab is eluted in the reaction liquid holding chamber 11, the staff places a sampling end of the sampling swab downward into the inner tube 2, and then places the inner tube 2 into the outer tube 1. The squeezing part 21 squeezes out the liquid in the reaction liquid holding chamber 11, and the liquid enters the inner tube 2 and the gap between the inner tube 2 and the outer tube 1 along the guide channel 22 and the overflow port 75. The liquid entering the inner tube 2 immerses the sampling end of the sampling swab, preventing detection errors caused by incomplete elution of the sampling swab and improving detection accuracy.

### Embodiment 3

Based on Embodiment 1, the present disclosure provides a sampling detection device with the inner tube 2 being a split structure.

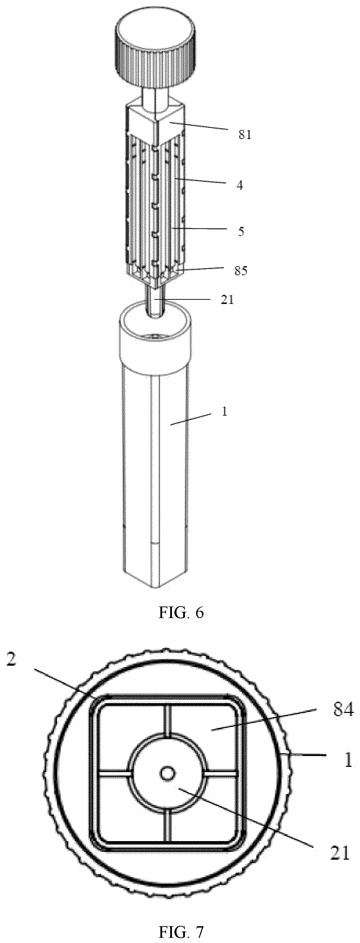

As shown in FIGS. 5, 6, and 7, the test strip mounting part 23 includes multiple mounting side plates 81. Each two adjacent mounting side plates 81 are connected in a clamping manner. The test strip mounting part 23 can have a regular shape such as rectangular and cylindrical, and can also have an irregular shape. In a feasible implementation, the test strip mounting part 23 is rectangular. The test strip mounting part 23 includes four mounting side plates 81. There are two test strip mounting positions 4 on an outer side wall of each mounting side plate 81. Each mounting side plate 81 includes one side provided with clamping element 82 and the other side provided with a clamping slot 83. The clamping element 82 of each mounting side plate 81 is connected in a clamping manner to the clamping slot 83 of one adjacent mounting side plate 81, thereby achieving the overall assembly of the test strip mounting part 23.

Furthermore, the bottom of each of the mounting side plates 81 is integrally formed with holding element 84. The four holding elements 84 at the bottom of the test strip mounting part 23 cooperate to hold the squeezing part 21. For example, the squeezing part 21 is cylindrical, and a contact part between the holding elements 84 and the squeezing part 21 is a quarter arc with a same curvature as the squeezing part 21. Therefore, the four holding elements 84 cooperate to stably hole the squeezing part 21, achieving the assembly of the inner tube 2. A side of the test strip mounting position 4 adjacent to the squeezing part 21 is provided with second communicating hole 85. The second communicating hole 85 communicates the test strip mounting position 4 with the internal space of the inner tube 2.

In the description of the present application, it needs to be understood the orientation or positional relationships indicated by terms, such as "up", "down", "front", "rear", "left", "right", "vertical", "horizontal", "top", "bottom", "inside", and "outside", are based on the orientation or positional relationship shown in the drawings, are merely for facilitating the description of the present application and simplifying the description, rather than indicating or implying that an apparatus or element referred to must have a particular orientation or be constructed and operated in a particular orientation, and therefore shall not be interpreted as limiting the present application.

Compared with the prior art, the present disclosure has the following beneficial effects:
1. In the present disclosure, multiple test strips are arranged at multiple test strip mounting positions on the outer wall of the inner tube, and the reaction liquid is placed inside the outer tube. After sampling, the sample is added into the reaction liquid holding chamber and thoroughly mixed in the reaction liquid holding chamber. Then, the inner tube is placed into the outer tube. The squeezing part of the inner tube squeezes the liquid in the reaction liquid holding chamber into the main body holding chamber, and the liquid immerses the chromatography parts of the multiple test strips. After a certain period of time, the results are determined, thereby completing the multi-target detection. The present disclosure features simple structure, convenient operation, and high batch detection efficiency.
2. In the present disclosure, the squeezing part is provided with a guide channel, which facilitates the flow of the liquid from the reaction liquid holding chamber into the main body holding chamber when the squeezing part extends into the reaction liquid holding chamber. The squeezing part cooperates with the reaction liquid holding chamber to efficiently utilize the reaction liquid, reducing ineffective liquid and improving sample utilization.
3. In the present disclosure, the inner tube is designed as a hollow structure, and the upper end of the inner tube is open. When multi-target detection is conducted, the staff places the sampling instrument (such as sampling swab) into the inner tube. The entire detection process is completed in a sealed environment, effectively preventing contamination of the environment caused by residual samples in the reaction liquid holding chamber and sampling equipment, enhancing environmental safety, and reducing the risk of infection for operators. The liquid entering the inner tube immerses the sampling end of the sampling instrument, preventing detection errors caused by incomplete elution of the sampling instrument and improving detection accuracy.

The specific embodiments of the present disclosure are described above. It should be understood that the present disclosure is not limited to the above specific implementations, and a person skilled in the art can make various variations or modifications within the scope of the claims without affecting the essence of the present disclosure. The embodiments of the present disclosure and features in the embodiments may be arbitrarily combined with each other in a non-conflicting situation.

## Claims

1. A sampling detection device, comprising an outer tube (1), an inner tube (2), and a tube cover (3), wherein the outer tube (1) comprises a reaction liquid holding chamber (11) and a main body holding chamber (12) arranged in sequence; the reaction liquid holding chamber (11) is communicated with the main body holding chamber (12); an end of the main body holding chamber (12) away from the reaction liquid holding chamber (11) is open; and a side wall of the main body holding chamber (12) comprises a transparent structure;
the inner tube (2) comprises a squeezing part (21) and a test strip mounting part (23) connected in sequence; and a peripheral side of the test strip mounting part (23) is provided with a plurality of test strip mounting positions (4);
the outer tube (1) is configured to hold the inner tube (2); the test strip mounting part (23) is located inside the main body holding chamber (12); the squeezing part (21) is inserted into the reaction liquid holding chamber (11); and the squeezing part (21) cooperates with an inner wall of the reaction liquid holding chamber (11) to form a gap that allows a reaction liquid to pass through; and
the tube cover (3) is detachably connected to the outer tube (1); and the tube cover (3) is configured to close or open an open side of the main body holding chamber (12).

2. The sampling detection device according to claim 1, wherein an outer wall of the squeezing part (21) is provided with a guide channel (22); and the guide channel (22) extends from an end of the squeezing part (21) away from the test strip mounting part (23) towards the test strip mounting part (23).

3. The sampling detection device according to claim 1, wherein the test strip mounting positions (4) are recessed on an outer wall of the test strip mounting part (23), and an opening edge of each of the test strip mounting positions (4) is provided with a limit stopper (5).

4. The sampling detection device according to claim 1, wherein a pressing plate (6) is provided in the tube cover (3); and after the tube cover (3) is provided in place on the outer tube (1), the pressing plate (6) abuts against the inner tube (2).

5. The sampling detection device according to claim 1, wherein the inner tube (2) comprises a hollow structure; an upper end of the inner tube (2) is open; and the inner tube (2) comprises an integrated structure or a split structure.

6. The sampling detection device according to claim 5, wherein the test strip mounting part (23) comprises a mounting body (71) and a connecting base (72); the mounting body (71) and the connecting base (72) are connected in an inserting manner; and an end of the connecting base (72) away from the mounting body (71) is securely connected to the squeezing part (21).

7. The sampling detection device according to claim 6, wherein an outer wall of the squeezing part (21) is provided with a guide channel (22); an end of the connecting base (72) adjacent to the squeezing part (21) is provided with an overflow port (75); and the overflow port (75) communicates the guide channel (22) with an internal space of the inner tube (2); and
a side of the test strip mounting position (4) located at the connecting base (72) is provided with a first communicating hole (76); and the first communicating hole (76) communicates the test strip mounting position (4) with the internal space of the inner tube (2).

8. The sampling detection device according to claim 5, wherein the test strip mounting part (23) comprises a plurality of mounting side plates (81); and each two adjacent mounting side plates (81) are connected in a clamping manner.

9. The sampling detection device according to claim 8, wherein a bottom of each of the mounting side plates (81) is provided with a holding element (84); and a plurality of holding elements (84) at a bottom of the test strip mounting part (23) cooperate to hold the squeezing part (21).

10. The sampling detection device according to claim 9, wherein a side of the test strip mounting position (4) adjacent to the squeezing part (21) is provided with a second communicating hole (85); and the second communicating hole (85) communicates the test strip mounting position (4) with an internal space of the inner tube (2).
